# EUROPEAN PATENT APPLICATION

(11) **EP 4 759 812 A1**
(43) Date of publication of application: **17.06.2026**
(21) Application number: 24850989.5
(22) Date of filing: 06.08.2024
(51) Int. Cl.: C07D 487/04, A61K 31/4985, A61P 35/00, A61P 37/00

(54) **CRYSTAL FORM VI OF ACRYLAMIDE COMPOUND, AND PREPARATION METHOD THEREFOR AND USE THEREOF**

(30) Priority: 07.08.2023 WO PCT/CN2023/111497
(71) Applicant: Guangzhou Lupeng Pharmaceutical Co., Ltd., Guangzhou, Guangdong 510670 (CN)
(72) Inventor: YE, Huiqing, Guangzhou, Guangdong 510700 (CN); ZHAN, Ningxin, Guangzhou, Guangdong 510700 (CN); WU, Chun, Guangzhou, Guangdong 510700 (CN); XU, Xuesong, Guangzhou, Guangdong 510700 (CN)
(74) Representative: Marks & Clerk LLP
(86) International application number: PCT/CN2024/110036
(87) International publication number: WO 2025/031344

(57) **Abstract**

Provided is a crystal form VI of an acrylamide compound. The acrylamide compound is an (S)-N-(5-((6-(7,7-dimethyl-1-oxo-1,3,4,6,7,8-hexahydro-2H-cyclopentane[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)5-phenyl)acrylamide compound, and the X-ray powder diffraction pattern of the crystal form VI has diffraction peaks at the following 2θ angle values: 8.3±0.2°, 13.2±0.2° and 18.9±0.2°. The crystal form VI has the advantages of good stability and low hygroscopicity, and is suitable for industrial production of bulk drugs and formulation development of preparations.

## Description

### Technical Field

The present invention pertains to the technical field of research and development of small-molecule chemical drugs, and more particularly, relates to a crystal form VI of an acrylamide compound, and a preparation method therefor and use thereof.

### Background Art

Bruton's tyrosine kinase (BTK) is a non-receptor protein kinase of the Tec family, which is expressed in most hematopoietic cells such as B cells, mast cells, and macrophages, but not in T cells, natural killer cells, and plasma cells [Smith, C.I. et al. Journal of Immunology (1994), 152(2), 557-65]. BTK is a critical part of the BCR and FcR signaling pathways, and targeted inhibition of BTK represents a novel approach for the treatment of many different human diseases, such as B-cell malignancies, autoimmune diseases, and inflammatory disorders [Uckun, Fatih M. et al., Anti-Cancer Agents in Medicinal Chemistry (2007); Shinohara et al., Cell 132 (2008) pp. 794-806; Pan, Zhengying, Drug News & Perspectives (2008), 21(7); 7(6), 624-632; Gilfillan et al., Immunological Reviews 288 (2009) pp. 149-169; Davis et al., Nature, 463 (2010) pp. 88-94].

Covalent Bruton's tyrosine kinase (BTK) inhibitors, including ibrutinib and acalabrutinib, have changed the treatment method for some BTK-dependent B-cell malignancies, wherein the B-cell malignancies include chronic lymphocytic leukemia, Waldenstrom macroglobulinemia, mantle cell lymphoma, and marginal zone lymphoma. Despite the impressive clinical responses of ibrutinib in B-cell malignancies, cases of primary and secondary resistance still occur, in which prognosis is poor and treatment options are limited. The majority of CLL patients who develop resistance to irreversible BTK inhibitors, such as ibrutinib, have the BTK-C481S mutation. It has been reported that 80% of patients with relapsed CLL will have the C481S mutation [Maddocks KJ, et al. JAMA Oncol. 2015; 1: 80-87]. Another research group at Ohio State University reported in the Journal of Clinical Oncology [Vol. 35, No. 13, 2017, p. 1437] that by year 4, approximately 20% of patients using ibrutinib had experienced clinical progression. 85% of these relapsed patients acquired the C481S mutation. Furthermore, these mutations were detected within an average of nine months prior to relapse.

Although BTK inhibitors such as ibrutinib and ACP-196 have made significant contributions to the art, there remains a strong need to continue developing highly potent and

selective BTK inhibitors that can not only irreversibly inhibit WT BTK but also reversibly inhibit the C481S mutant BTK.

Patent Publication No. CN111741959A discloses a small-molecule BTK inhibitor for treatment of a neoplastic disease or tumor, an autoimmune disease, and an inflammatory disorder, and specifically discloses a representative compound:

Chinese chemical name: (S)-N-(5-((6-(2-(7,7- 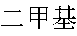 -1- 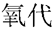 -1,3,4,6,7,8- 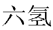 -2H- 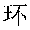 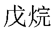 [4,5] 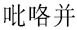 [1,2-a] 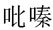 -2- 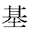 )-3-( 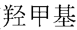 ) 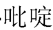 -4- 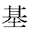 )-4- 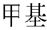 -3- 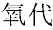 -3,4- 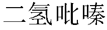 -2- 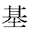 ) 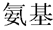 )-2-(2- 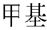 -4-( 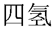 -2H- 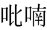 -4- 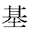 ) 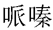 -1- 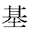 ) 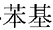 ) 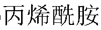 .

English chemical name: (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide.

For solid substances, the influence of various factors such as molecular structure configuration, conformation, molecular arrangement, molecular forces, and co-crystal substances leads to different spatial arrangements of the molecular lattice, resulting in the formation of two or more different crystal structures. This phenomenon is known as the "polymorphism phenomenon" or "polymorphism." The "polymorphism phenomenon" is widespread in solid drugs. Different crystal forms of the same drug may exhibit differences in physicochemical properties, such as appearance, density, hardness, melting point, solubility, stability, dissolution, dissolution rate, and bioavailability, thereby affecting the uniformity, bioavailability, efficacy, and safety of the drug to varying degrees. Furthermore, the existence forms and quantities of polymorphic compounds are unpredictable, and the physicochemical stability, processability, bioavailability, efficacy, and safety of different crystal forms are not obvious.

Therefore, during the research and development of new drugs, it is necessary to comprehensively screen the crystalline and amorphous forms of drug compounds, taking multiple factors into consideration. In particular, for the above compounds used as small-molecule BTK inhibitors, their hygroscopicity, stability, and morphology require further improvement. Therefore, developing crystalline forms of the compound or a salt thereof that may possess medicinal value to improve characteristics such as the stability of the compound holds potential medicinal and clinical value.

### Summary of the Invention

In view of the above problems in the prior art, a primary objective of the present invention is to provide a crystal form VI of an acrylamide compound, which has the advantages of good stability and low hygroscopicity.

The above objective of the present invention is achieved by the following technical solutions:

The above acrylamide compound, whose Chinese name is: (S)-N-(5-((6-(2-(7,7- 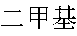 -1- 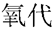 -1,3,4,6,7,8- 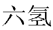 -2H- 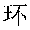 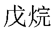 [4,5] 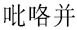 [1,2-a] 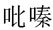 -2- 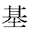 )-3-( 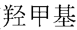 ) 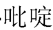 -4 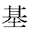 )-4- 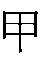 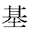 -3- 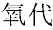 -3,4- 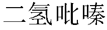 -2- 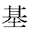 ) 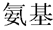 )-2-(2- 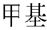 -4-( 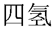 -2H- 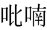 -4- 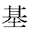 ) 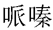 -1- 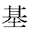 ) 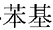 ) 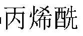 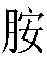 , is a known inhibitor of BTK and a mutant thereof.

A first objective of the present application is to provide a crystal form VI of the above compound, where an X-ray powder diffraction pattern of the crystal form VI includes diffraction peaks at the following 2θ angle values: 8.3±0.2°, 13.2±0.2°, 13.6±0.2°, and 18.9±0.2°;

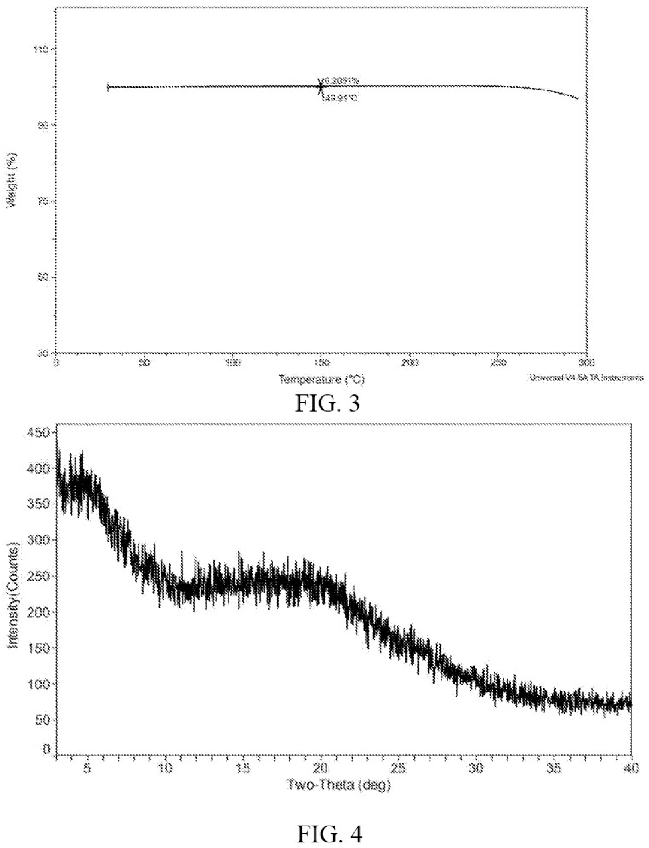

Patent CN111741959A discloses an (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide compound. An X-ray powder diffraction pattern of an amorphous form of the above compound is shown in FIG. 4. The amorphous form presents some difficulties in drug formulation and development, such as low stability, high hygroscopicity, and relatively poor powder properties, which are not conducive to drug development.

The inventors have discovered a stable crystalline form of the above compound, crystal form VI, through extensive experimental research. The crystal form VI provided by the present invention has advantages such as good stability, low hygroscopicity, and good crystal morphology. Suspensions formed by the crystal form VI in pure water and buffer solutions of different pH values have extremely excellent stability. Furthermore, the crystal form VI showed no change in crystal form and no significant changes in related substances after storage under the conditions of 60°C for 30 days, 25°C/60% RH for 24 months, and 40°C/75% RH for 6 months, indicating relatively good physical and chemical stability. DVS results of the crystal form VI show that it has a weight gain of 0.79% at 80% relative humidity and 1.34% at 95% relative humidity, indicating slight hygroscopicity. The crystals of the crystal form VI are long rod-shaped and exhibit relatively good dispersibility and flowability.

In some embodiments, the X-ray powder diffraction pattern of the crystal form VI includes diffraction peaks at the following 2θ angle values: 8.3±0.2°, 11.3±0.2°, 13.2±0.2°, 13.6±0.2°, 18.9±0.2°, and 21.5±0.2°;

In some embodiments, the X-ray powder diffraction pattern of the crystal form VI includes diffraction peaks at the following 2θ angle values: 4.3±0.2°, 8.3±0.2°, 11.3±0.2°, 13.2±0.2°, 13.6±0.2°, 17.8±0.2°, 18.9±0.2°, and 21.5±0.2°.

In some embodiments, the X-ray powder diffraction pattern of the crystal form VI includes diffraction peaks at the following 2θ angle values: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°.

In some embodiments, characteristic peak information on a partial X-ray powder diffraction pattern of the crystal form VI is as shown in Table 1 below.

**Table 1**

| 2θ value (°) | d-spacing value (Å) | Intensity (%) |
|---|---|---|
| 4.3 | 20.34 | 15.2 |
| 6.9 | 12.72 | 12.2 |
| 8.3 | 10.69 | 100 |
| 9.7 | 9.15 | 15.2 |
| 11.3 | 7.82 | 42.7 |
| 13.2 | 6.72 | 57.2 |
| 13.6 | 6.49 | 40.2 |
| 14.5 | 6.09 | 13.1 |
| 17.8 | 4.97 | 22.7 |
| 18.9 | 4.68 | 80.6 |
| 20.1 | 4.41 | 20.2 |
| 21.5 | 4.14 | 41.8 |

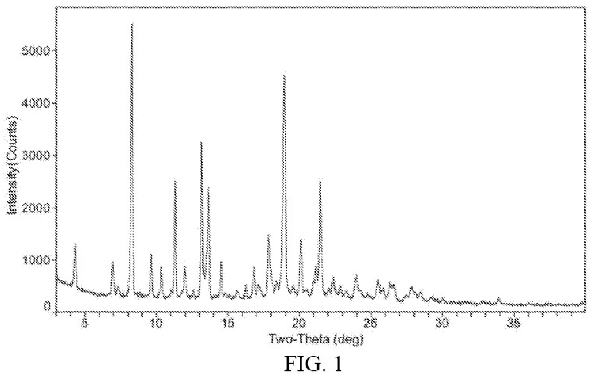

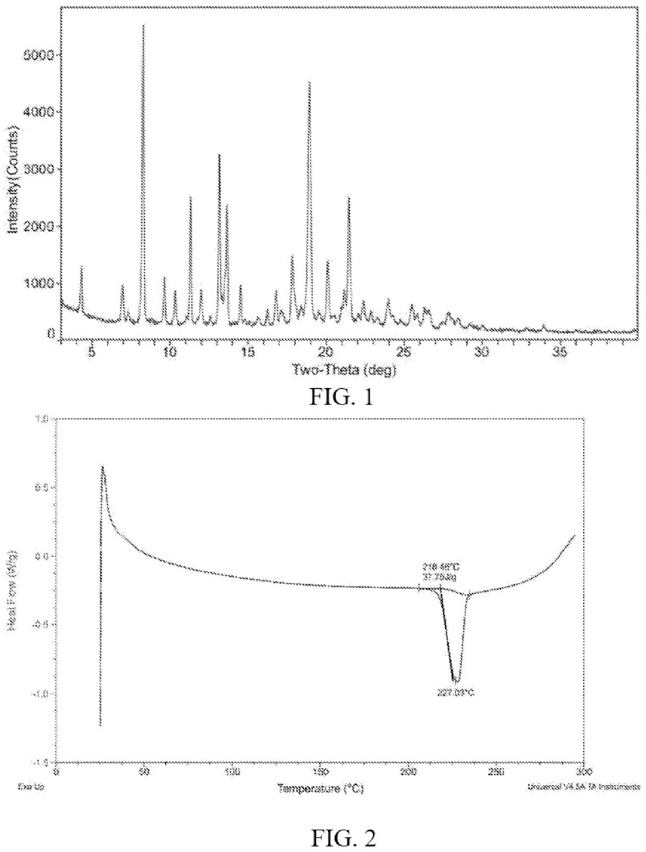

In some embodiments, the crystal form VI has an X-ray powder diffraction pattern substantially consistent with that shown in FIG. 1.

In some embodiments, the crystal form VI is an anhydrate.

In some embodiments, the crystal form VI has a purity of >85%.

In some embodiments, the crystal form VI has a purity of >95%.

In some embodiments, the crystal form VI has a purity of >99%.

In some embodiments, the crystal form VI has a melting point onset peak at 218.5±3°C and an endothermic melting peak value at 227.0±3°C.

In some embodiments, the crystal form VI has a DSC pattern substantially consistent with that shown in FIG. 2.

In some embodiments, a thermogravimetric analysis curve of the crystal form VI has a weight loss of 0.21%±0.1% at 30-150°C. The crystal form VI has no significant stepwise weight loss between 30°C and 150°C.

In some embodiments, the crystal form VI has a TGA pattern substantially consistent with that shown in FIG. 3.

Further, the present invention further provides a novel crystal form V of the above compound.

An X-ray powder diffraction pattern of the crystal form V of the above compound provided by the present invention includes diffraction peaks at the following 2θ angle values: 9.2±0.2°, 15.4±0.2° and 21.5±0.2°.

In some embodiments, the X-ray powder diffraction pattern of the crystal form V includes diffraction peaks at the following 2θ angle values: 9.2±0.2°, 15.4±0.2°, 19.9±0.2°, 20.5±0.2°, 21.5±0.2°, 22.2±0.2°, and 25.0±0.2°.

In some embodiments, the X-ray powder diffraction pattern of the crystal form V includes diffraction peaks at the following 2θ angle values: 4.1±0.2°, 9.2±0.2°, 11.7±0.2°, 14.8±0.2°, 15.4±0.2°, 18.2±0.2°, 19.9±0.2°, 20.5±0.2°, 21.5±0.2°, 22.2±0.2°, 25.0±0.2°, and 27.7±0.2°.

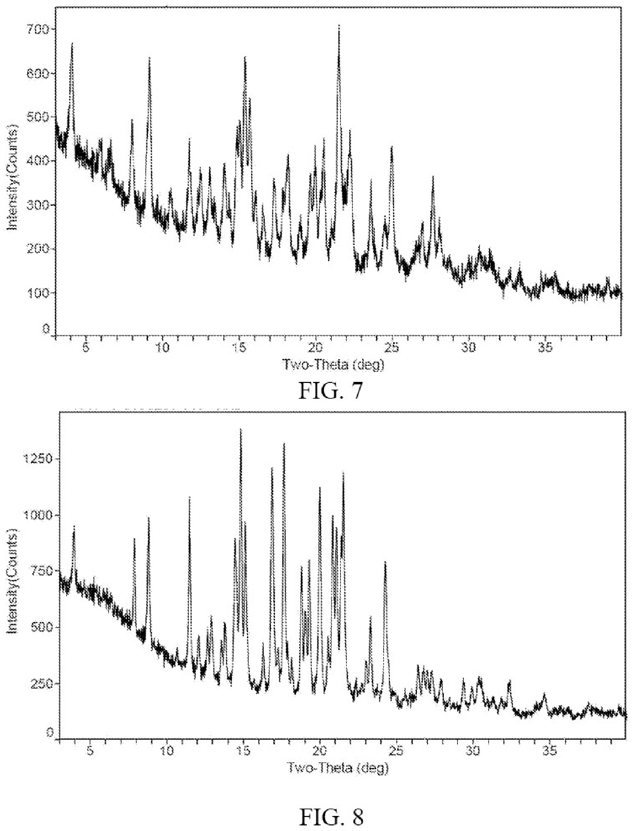

In some embodiments, the crystal form V has an X-ray powder diffraction pattern substantially consistent with that shown in FIG. 7.

In some embodiments, the crystal form V is a hydrate.

Further, the present invention further provides a novel crystal form XIII of the above compound.

An X-ray powder diffraction pattern of the crystal form XIII of the above compound provided by the present invention includes diffraction peaks at the following 2θ angle values: 14.8±0.2°, 16.9±0.2°, 17.6±0.2°, and 21.5±0.2°.

In some embodiments, the X-ray powder diffraction pattern of the crystal form XIII includes diffraction peaks at the following 2θ angle values: 11.5±0.2°, 14.8±0.2°, 16.9±0.2°, 17.6±0.2°, 20.0±0.2°, 20.8±0.2°, 21.5±0.2°, and 24.3±0.2°.

In some embodiments, the X-ray powder diffraction pattern of the crystal form XIII includes diffraction peaks at the following 2θ angle values: 11.5±0.2°, 14.5±0.2°, 14.8±0.2°, 15.1±0.2°, 16.9±0.2°, 17.6±0.2°, 18.8±0.2°, 19.3±0.2°, 20.0±0.2°, 20.8±0.2°, 21.5±0.2°, and 24.3±0.2°.

In some embodiments, the crystal form XIII has an X-ray powder diffraction pattern substantially consistent with that shown in FIG. 8.

In some embodiments, the crystal form XIII is a hydrate.

In some embodiments, a differential scanning calorimetry curve of the crystal form XIII has an endothermic melting peak at 129.0±3°C and 261.5±3°C, respectively.

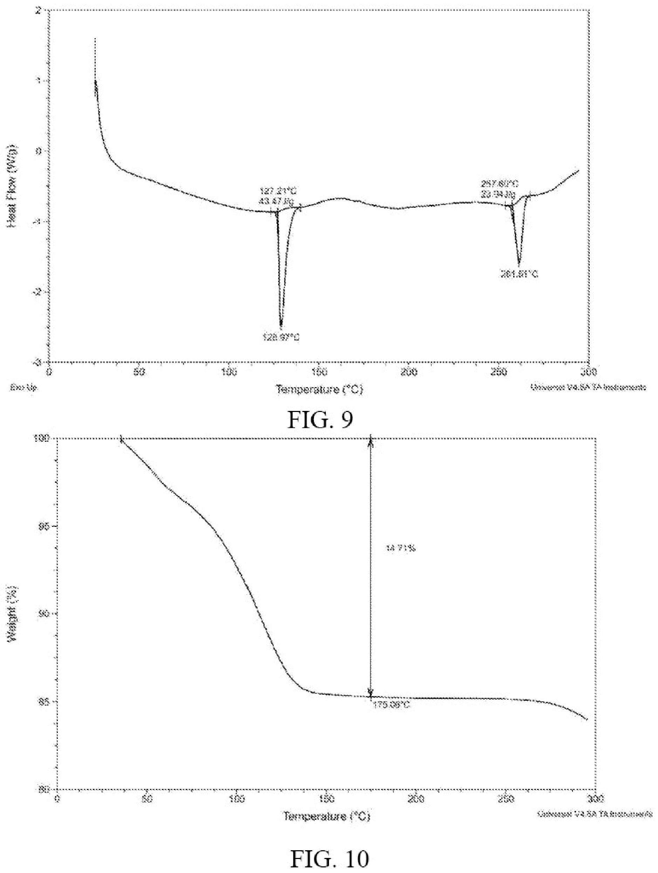

In some embodiments, the crystal form XIII has a DSC pattern substantially consistent with that shown in FIG. 9.

In some embodiments, a thermogravimetric analysis curve of the crystal form XIII has a weight loss of about 14.71% between 30°C and 170°C.

In some embodiments, the crystal form XIII has a TGA pattern substantially consistent with that shown in FIG. 10.

Further, a second objective of the present invention is to provide a preparation method for the crystal form VI of the compound, including: adding the compound into a first solvent to dissolve the compound and form a clear solution, and then treating the clear solution using a crystallization process to obtain the crystal form VI; where the first solvent is selected from one or more of alcohol-based, carboxylic ester-based, nitrile-based, ketone-based, amide-based, sulfone-based, sulfoxide-based, carboxylic acid-based, nitro compound, pyridine-based, furan-based, alkylbenzene-based, and halogenated hydrocarbon-based polar solvents; or
adding the compound into a second solvent for slurrying, cooling, and separating a precipitated crystal form VI; wherein the second solvent is selected from one or more of alcohol-based, ether-based, alkane-based, carboxylic ester-based, nitrile-based, ketone-based, or alkylbenzene-based solvents.

The preparation method provided by the present invention has a simple process, green and environmentally friendly solvents, and a high yield, and is suitable for industrial scale-up production.

In some embodiments, the crystallization process comprises performing crystallization by volatilization on the clear solution to slowly volatilize the first solvent and obtain the crystal form VI.

When crystallization is performed by adopting the crystallization by volatilization as described above, in some embodiments, the first solvent is selected from ketone-based and/or carboxylic ester-based polar solvents; more specifically, the first solvent is selected from a ketone-based polar solvent containing 3-6 carbon atoms and/or a carboxylic ester-based polar solvent containing 3-10 carbon atoms; and more specifically, the first solvent is a mixed solvent of acetone and isopropyl acetate.

In some embodiments, when the first solvent is a mixed solvent of acetone and isopropyl acetate, a volume-to-mass ratio of the acetone to the compound is 40-60 mL/g, and a volume-to-mass ratio of the isopropyl acetate to the compound is 5-25 mL/g.

In some embodiments, the crystallization process comprises performing crystallization by evaporation on the clear solution to obtain the crystal form VI.

When crystallization is performed by adopting the crystallization by evaporation as described above, in some embodiments, the first solvent is selected from halogenated hydrocarbon-based and/or ketone-based polar solvents; more specifically, the first solvent is selected from a halogenated hydrocarbon-based polar solvent containing 1-6 carbon atoms and/or a ketone-based polar solvent containing 3-6 carbon atoms; and more specifically, the first solvent is dichloromethane and acetone.

In some embodiments, when the first solvent is dichloromethane and acetone, a volume-to-mass ratio of the dichloromethane to the compound is 1-50 mL/g, and a volume-to-mass ratio of the acetone to the compound is 1-100 mL/g; and more specifically, a volume-to-mass ratio of the dichloromethane to the compound is 1-30 mL/g, and a volume-to-mass ratio of the acetone to the compound is 1-50 mL/g.

In some embodiments, the compound is added to the dichloromethane and dissolved at room temperature to form a clear solution. The clear solution is concentrated under reduced pressure to 1/6 to 1/4 of a volume. The acetone is slowly added dropwise at 35-45°C to precipitate the crystal form VI. The mixture is concentrated under reduced pressure to 2/5 to 2/3 of a volume, cooled to 10-25°C, stirred, and subjected to suction filtration to obtain the crystal form VI.

In some embodiments, the crystallization process comprises adding a poor solvent to the clear solution to precipitate the crystal form VI.

When crystallization is performed by adopting the poor solvent as described above, the first solvent is selected from one or more of amide-based, halogenated hydrocarbon-based, sulfoxide-based, and furan-based polar solvents; and the poor solvent is selected from one or more of an alcohol, a carboxylic ester, a nitrile, a ketone, an alkane, an ether, a nitro compound, and an alkylbenzene. The crystal form VI has relatively good solubility in the above first solvent. Therefore, the above first solvent can be used as a good solvent for the crystal form VI. In contrast, the crystal form VI has relatively poor solubility in the above poor solvent.

In some embodiments, the first solvent is selected from a halogenated hydrocarbon-based polar solvent, and the poor solvent is selected from an ether-based solvent; more specifically, the first solvent is selected from a halogenated hydrocarbon-based polar solvent containing 1-6 carbon atoms, and the poor solvent is selected from an ether-based solvent containing 2-6 carbon atoms; and more specifically, the first solvent is dichloromethane, and the poor solvent is methyl tert-butyl ether.

In some embodiments, a volume-to-mass ratio of the first solvent to the compound is 1-50 mL/g; and a volume-to-mass ratio of the poor solvent to the compound is 1-80 mL/g; and more specifically, a volume-to-mass ratio of the first solvent to the compound is 3-40 mL/g; a volume-to-mass ratio of the poor solvent to the compound is 3-50 mL/g.

When crystallization is performed by adopting a slurrying method as described above, in some embodiments, the second solvent is selected from ketone-based and/or nitrile-based solvents; more specifically, the second solvent is selected from a ketone-based solvent containing 3-6 carbon atoms and/or a nitrile-based solvent containing 2-6 carbon atoms; and more specifically, the second solvent is acetonitrile or acetone.

In some embodiments, a volume-to-mass ratio of the second solvent to the compound is 1-30 mL/g; and more specifically, a volume-to-mass ratio is 5-20 mL/g.

In some embodiments, a temperature of the slurrying is 0-80°C; and more specifically, a temperature of the slurrying is 10-50°C.

In some embodiments, a time of the slurrying is 1-120 h; and more specifically, a time of the slurrying is 4-20 h.

In some embodiments, a process of the slurrying further includes stirring, for example, stirring for 1-24 hours, or longer; and more specifically, stirring for at least 4 hours, such as 5 hours and the like.

Further, a third objective of the present invention is to provide a pharmaceutical composition, including a therapeutically effective amount of the crystal form VI of the above compound and a pharmaceutically acceptable carrier or adjuvant.

In some embodiments, the adjuvant includes, but is not limited to, a filler, an excipient, a binder, a disintegrant, a surfactant, a pH regulator, a glidant, a lubricant, or other suitable additives, and the like.

In some embodiments, a preparation method for the pharmaceutical composition is filling the pharmaceutical composition containing the crystal form VI in a form of a powder, a granule, a pellet, or the like into a capsule or preparing it into a tablet; and more specifically, the granule may be prepared by wet granulation or dry granulation and other suitable granulation methods.

In some embodiments, the crystal form VI may account for 1-99% by weight of the pharmaceutical composition; more specifically, 1-60% by weight; more specifically, 1-50% by weight; and more specifically, 1-30% by weight.

Further, a fourth objective of the present invention is to provide an application of the crystal form VI of the compound or the above pharmaceutical composition in preparation of a medicament for preventing and/or treating a neoplastic disease or tumor, an autoimmune disease, and an inflammatory disorder.

In some embodiments, the autoimmune disease and the inflammatory disorder are pemphigus vulgaris, rheumatoid arthritis, asthma, multiple sclerosis, systemic lupus erythematosus, or allergy.

In some embodiments, the neoplastic disease or tumor is a B-cell malignancy or a solid tumor.

In some embodiments, the neoplastic disease or tumor is chronic lymphocytic leukemia (CLL), small lymphocytic lymphoma (SLL), mantle cell lymphoma (MCL), diffuse large B-cell lymphoma (DLBCL), or multiple myeloma (MM).

Further, a fifth objective of the present invention is to provide a method for preventing and/or treating a neoplastic disease or tumor, an autoimmune disease, and an inflammatory disorder, including administering a therapeutically effective amount of the crystal form VI of the compound to a subject.

In some embodiments, an administration dosage of the crystal form VI is 0.5-200 mg/day; in some embodiments, an administration dosage of the crystal form VI is 1-100 mg/day; and in some embodiments, an administration dosage of the crystal form VI is 5-50 mg/day. An administration frequency for the above is one to three times per day.

In some embodiments, the crystal form VI or the pharmaceutical composition of the present invention may be preferably administered orally. More specifically, oral administration may be preferably performed in a form of a capsule or a tablet.

Compared with the prior art, the present invention has the following beneficial effects:
The crystal form VI provided by the present invention has advantages such as good stability, low hygroscopicity, and good crystal morphology. The crystal form VI has extremely excellent stability in suspensions formed in pure water and buffer solutions with different pH values. Furthermore, the crystal form VI showed no change in crystal form and no significant changes in related substances after storage under the conditions of 60°C for 30 days, 25°C/60% RH for 24 months, and 40°C/75% RH for 6 months, indicating relatively good physical and chemical stability. DVS results of the crystal form VI show that it has a weight gain of 0.79% at 80% relative humidity and 1.34% at 95% relative humidity, indicating slight hygroscopicity. The crystals of the crystal form VI are long rod-shaped and exhibit relatively good dispersibility and flowability. Furthermore, a preparation of the crystal form VI shows good exposure in animal PK, meeting the requirements for *in vivo* drug exposure. The crystal form VI of the compound provided by the present invention is beneficial to storage of bulk drugs and preparation of preparations, is beneficial to improvement of drug quality and stability, and is suitable for industrial production of bulk drugs and formulation development of preparations.

### Brief Description of the Drawings

FIG. 1 is an X-ray powder diffraction (XRPD) pattern of a crystal form VI.
FIG. 2 is a differential scanning calorimetry (DSC) pattern of a crystal form VI.
FIG. 3 is a thermogravimetric analysis (TGA) diagram of a crystal form VI.
FIG. 4 is an X-ray powder diffraction (XRPD) pattern of an amorphous form.
FIG. 5 is a proton nuclear magnetic resonance spectrum of a compound.
FIG. 6 is an infrared spectrum of a compound.
FIG. 7 is an X-ray powder diffraction (XRPD) pattern of a crystal form V.
FIG. 8 is an X-ray powder diffraction (XRPD) pattern of a crystal form XIII.
FIG. 9 is a differential scanning calorimetry (DSC) pattern of a crystal form XIII.
FIG. 10 is a thermogravimetric analysis (TGA) diagram of a crystal form XIII.
FIG. 11 is a dynamic vapor sorption (DVS) test plot of a crystal form VI.
FIG. 12 is a dynamic vapor sorption (DVS) test plot of an amorphous form.
FIG. 13 is a dynamic vapor sorption (DVS) test plot of a crystal form XIII.
FIG. 14 is an influencing factor stability test plot of a crystal form VI.
FIG. 15 is a polarizing light microscope (400X) image of a crystal form VI.
FIG. 16 is a polarizing light microscope (100X) image of an amorphous form.
FIG. 17 is a polarizing light microscope (400X) image of a crystal form XIII.

### Detailed Description of Embodiments

In the following description, certain specific details are set forth to provide a thorough understanding of various embodiments of the present invention. However, those skilled in the art will understand that the present invention may be practiced without these details. The description of various embodiments below is made with the understanding that the present disclosure is to be considered as an exemplification of the claimed subject matter, and is not intended to limit the appended claims to the specific embodiments illustrated. Headings used throughout the present disclosure are merely to provide convenience and are not to be construed as limiting the claims in any way. Embodiments described under any heading may be combined with embodiments described under any other heading.

In the present invention, the crystal form can be determined by various technical means, such as X-ray powder diffraction (XRPD), infrared absorption spectroscopy (IR), a melting point method, differential scanning calorimetry (DSC), thermogravimetric analysis (TGA), a nuclear magnetic resonance method, Raman spectroscopy, X-ray single crystal diffraction, solution calorimetry, scanning electron microscopy (SEM), quantitative analysis, solubility and a dissolution rate, and the like.

In the present invention, X-ray powder diffraction (XRPD) can detect information such as changes in the crystal form, crystallinity, and crystal structure state, and is a common means for identifying the crystal form. The peak positions in an XRPD pattern depend primarily on the structure of the crystal form and are relatively insensitive to experimental details, whereas the relative peak heights of the pattern depend on many factors related to sample preparation and instrument geometry. Accordingly, in some embodiments, the crystal form of the present invention is characterized by an XRPD pattern having certain peak positions, which is substantially as shown in the XRPD pattern provided in the drawings of the present invention. Meanwhile, the measurement of 2θ in the XRPD pattern may have experimental errors. The measurement of 2θ in the XRPD pattern may vary slightly between different instruments and different samples. Therefore, the values of 2θ cannot be regarded as absolute. According to the condition of the instrument used in the test of the present invention, the diffraction peak has an error margin of ±0.2°. In the present invention, the XRPD tests are performed using a Bruker D8 Advance model instrument with a copper target and Kα radiation at a wavelength of 1.54 Å (voltage: 40 kV, current: 40 mA).

In the present invention, differential scanning calorimetry (DSC) is a technique that measures the difference in energy between a sample and an inert reference (commonly α-Al₂O₃) as a function of temperature through program control involving continuous heating or cooling. The height of the melting peak in a DSC curve depends on many factors related to sample preparation and instrument geometry, whereas the peak position is relatively insensitive to experimental details. Accordingly, in some embodiments, the crystal form of the present invention is characterized by a DSC pattern having a characteristic peak position, which is substantially as shown in the DSC pattern provided in the drawings of the present invention. Meanwhile, a DSC pattern may have experimental errors. The peak positions and peak values of the DSC pattern may vary slightly between different instruments and different samples. Therefore, the values of the peak positions or peak values of the DSC endothermic peaks cannot be regarded as absolute. According to the condition of the instrument used in the test of the present invention, the melting peak has an error margin of ±3°C.

In the present invention, differential scanning calorimetry (DSC) can also be used to detect and analyze whether the crystal form has a crystal transformation or mixed crystal phenomena. Solids with the same chemical composition often form polymorphs with different crystal structures, also referred to as variants, under different thermodynamic conditions. This phenomenon is referred to as a polymorphism or polytypism phenomenon. When temperature and pressure conditions change, mutual transformation occurs between variants. This phenomenon is referred to as polymorphic transformation. Due to polymorphic transformation, the mechanical, electrical, magnetic, and other properties of the crystal undergo significant changes. When the temperature of polymorphic transformation is within the measurable range, this transformation process can be observed on a differential scanning calorimetry (DSC) pattern, which is characterized in that the DSC pattern has an exothermic peak reflecting this transformation process, and simultaneously has two or more endothermic peaks, which are characteristic endothermic peaks of different crystal forms before and after the transformation, respectively.

In the present invention, thermogravimetric analysis (TGA) is a technique that measures the change in mass of a substance as a function of temperature through program control. It is suitable for examining the loss of solvent in crystals or the processes of sample sublimation and decomposition, and can be used to infer the presence of crystal water or crystal solvents in the crystals. The mass change displayed in a TGA curve depends on many factors such as sample preparation and the instrument. The mass change detected by TGA varies slightly between different instruments and different samples. According to the condition of the instrument used in the test of the present invention, the mass change has an error margin of ±0.1%.

Furthermore, when referring to, for example, an XRPD pattern, a TGA diagram, a DSC pattern, a DVS plot, etc., the term "substantially consistent with FIG...." means that the pattern, diagram, or plot is not necessarily identical to that described herein, but falls within the limits of experimental error or deviation when considered by those of ordinary skill in the art.

In the present invention, the "first solvent" refers to one or more selected from alcohol-based, carboxylic ester-based, nitrile-based, ketone-based, amide-based, sulfone-based, sulfoxide-based, carboxylic acid-based, nitro compound, pyridine-based, furan-based, alkylbenzene-based, and halogenated hydrocarbon-based polar solvents; and more specifically, the first solvent includes, but is not limited to, methanol, ethanol, trifluoroethanol, propanol, isopropanol, butanol, tert-butanol, polyethylene glycol, ethyl acetate, isopropyl acetate, acetonitrile, acetone, butanone, methyl ethyl ketone, 1-methyl-2-pyrrolidone, N,N-dimethylacetamide, N,N-dimethylformamide, formamide, dimethyl sulfoxide, chloroform, dichloromethane, formic acid, acetic acid, nitromethane, pyridine, tetrahydrofuran, toluene, xylene, and a mixture formed by any combination thereof, and the like. The alkylbenzene is benzene in which hydrogen atoms are substituted by one or more alkanes containing 1-6 carbon atoms; and more specifically, the alkylbenzene is benzene in which hydrogen atoms are substituted by one or more methyl groups.

In the present invention, the "second solvent" refers to one or more selected from alcohol-based, ether-based, alkane-based, carboxylic ester-based, nitrile-based, ketone-based, or alkylbenzene-based solvents; and more specifically, the second solvent includes, but is not limited to, 1,4-dioxane, ethyl acetate, acetone, methyl ethyl ketone, 1-methyl-2-pyrrolidone, 2-propanone, and a mixture formed by any combination thereof, and the like. The alkylbenzene is benzene in which hydrogen atoms are substituted by one or more alkanes containing 1-6 carbon atoms; and more specifically, the alkylbenzene is benzene in which hydrogen atoms are substituted by one or more methyl groups.

In the present invention, the good solvent is selected from one or more of amide-based, halogenated hydrocarbon-based, sulfoxide-based, and furan-based polar solvents; and more specifically, the good solvent includes, but is not limited to, N,N-dimethylacetamide, N,N-dimethylformamide, chloroform, dichloromethane, dimethyl sulfoxide, tetrahydrofuran, and a mixture thereof, and the like.

In the present invention, the poor solvent is selected from one or more of an alcohol, a carboxylic ester, a nitrile, a ketone, an alkane, an ether, a nitro compound, and an alkylbenzene; and more specifically, the poor solvent includes, but is not limited to, isopropanol, isopropyl acetate, acetonitrile, acetone, butanone, methyl ethyl ketone, heptane, hexane, 1,4-dioxane, methyl tert-butyl ether, isopropyl ether, nitromethane, toluene, and a mixture thereof, and the like. The alkylbenzene is benzene in which hydrogen atoms are substituted by one or more alkanes containing 1-6 carbon atoms; and more specifically, the alkylbenzene is benzene in which hydrogen atoms are substituted by one or more methyl groups.

In the present invention, the "good solvent" refers to a solvent in which the solubility of the crystal form VI of the compound is greater than 1 g/L, greater than 2 g/L, greater than 3 g/L, greater than 4 g/L, greater than 5 g/L, greater than 6 g/L, greater than 7 g/L, greater than 8 g/L, greater than 9 g/L, greater than 10 g/L, greater than 15 g/L, greater than 20 g/L, greater than 30 g/L, greater than 40 g/L, greater than 50 g/L, greater than 60 g/L, greater than 70 g/L, greater than 80 g/L, greater than 90 g/L, or greater than 100 g/L. In certain embodiments, based on the solubility in the dissolution solvent, the solubility of the crystal form VI of the compound in the good solvent and the poor solvent differs by about 10%, 20%, 30%, 40%, 50%, 60%, 70%, 80%, or 90%. In some embodiments, the solubility of the crystal form VI of the compound in the good solvent is greater than its solubility in the poor solvent, and the solubility of the crystal form VI of the compound in the good solvent is about 10%, 20%, 30%, 40%, 50%, 60%, 70%, 80%, or 90% higher than the solubility in the poor solvent.

In the present invention, the "poor solvent" refers to a solvent capable of promoting solution supersaturation and/or crystallization. In some embodiments, the solubility of the crystal form VI of the compound in the poor solvent is less than 0.001 g/L, less than 0.1 g/L, less than 0.2 g/L, less than 0.3 g/L, less than 0.4 g/L, less than 0.5 g/L, less than 0.6 g/L, less than 0.7 g/L, less than 0.8 g/L, or less than 1 g/L.

A suitable pharmaceutical carrier includes water, various organic solvents, various inert diluents, and the like; and a suitable adjuvant includes a filler, an excipient, a binder, a disintegrant, a surfactant, a pH regulator, a glidant, a lubricant, or another suitable additive, and the like. If necessary, a pharmaceutical composition may contain various additives, such as a fragrance, a binder, and an excipient. For oral administration, a tablet and a capsule may contain various excipients; some examples of suitable excipients include lactose, glucose, sucrose, sorbitol, mannitol, starch, gum arabic, calcium phosphate, an alginate, tragacanth, gelatin, calcium silicate, microcrystalline cellulose, polyvinylpyrrolidone, cellulose, water, syrup, and methylcellulose; various fillers, such as lactose monohydrate and microcrystalline cellulose, and the like; various glidants, such as silicon dioxide; various disintegrants, such as croscarmellose sodium, starch, alginic acid, and some silicates; and various binders, such as povidone K30, sucrose, gelatin, and gum arabic; and a pH regulator, such as fumaric acid, and the like. Furthermore, a lubricant or an anti-adherent, including magnesium stearate and talc, is generally used in the production of tablets. The same types of solid components may also be used for formulating soft and hard gelatin capsules. When an aqueous suspension is required for oral administration, an active compound may be mixed in combination with various sweetening or flavoring agents, pigments, or dyes. If desired, various emulsifying agents may be used or suspending agents may be produced; and a diluent such as water, ethanol, propylene glycol, glycerol, or a combination thereof may be used.

In the present invention, the pharmaceutical composition may be administered in the following forms: orally in a form such as a capsule, a tablet, a pill, or a powder; as a sustained-release injection (such as a sterile solution, a suspension, or an emulsion); through a topical treatment form such as a paste, a cream, or an ointment; or through a suppository such as a suppository form; or through inhalation or insufflation. The pharmaceutical composition may be in a unit dosage form suitable for precise dosage application.

The pharmaceutical composition may be formulated in unit dosage form, each dosage containing from about 5 mg to about 100 mg, more usually from about 10 mg to about 30 mg, of an active ingredient. The term "unit dosage form" refers to physically discrete units suitable as unitary dosages for human subjects and other mammals, each unit containing a predetermined quantity of active material calculated to produce the desired therapeutic effect in association with a suitable pharmaceutical excipient.

The pharmaceutical composition for inhalation or insufflation includes a solution, a suspension, or a powder in a pharmaceutically acceptable aqueous or organic solvent, or a mixture thereof. The liquid or solid composition may contain a suitable pharmaceutically acceptable excipient as described above. In some embodiments, the composition is administered by an oral or nasal respiratory route to achieve a local or systemic effect. The composition may be nebulized by use of an inert gas. The nebulized solution may be breathed directly from a nebulizing device, or the nebulizing device may be connected to a face mask, a tent, or an intermittent positive pressure respirator. The solution, suspension, or powder composition may be administered orally or nasally from a device which delivers a formulation in an appropriate manner.

The amount of the crystal form or composition administered to a patient will depend on factors such as the subject of administration, the purpose of administration such as prevention or treatment, the condition of the patient, the mode of administration, and the like. In therapeutic applications, the composition may be administered to a patient already suffering from a disease in an amount sufficient to cure or at least partially arrest the symptoms of the disease and its complications. An effective dosage will depend on the disease condition being treated and the judgment of the attending clinician according to factors such as the severity of a disease, and the age, weight, and general condition of a patient.

The term "treatment" refers to (for example) preventing, inhibiting, and ameliorating a disease, a condition, or a disorder in an individual.

The phrase "therapeutically effective amount" refers to the amount of an active compound or pharmaceutical agent that elicits a biological or medical response in a tissue, a system, an animal, an individual, or a human that is being sought by a researcher, a veterinarian, a physician, or other clinicians.

### Embodiment 1

### 1. Preparation of Crystal Form VI by Dilution Crystallization in Dichloromethane/Methyl Tert-butyl Ether System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 5.0 g of the compound raw material was added to dichloromethane (20 mL) and heated to 40°C for complete dissolution to obtain a clear solution. Methyl tert-butyl ether (80 mL) was added dropwise to induce crystallization. The mixture was then slowly cooled to 0°C, stirred for 5 h, subjected to suction filtration, and dried to obtain 4.51 g of an off-white solid powder with a yield of 90.2%. XRPD measurement confirmed it as a crystal form VI.

### 2. Identification of Crystal Form VI

(1) The off-white solid powder prepared in step (2) above was identified by Bruker D8 Advance X-ray powder diffraction (XRPD) analysis: Cu-Kα radiation was used, and it had characteristic peaks represented by the angle 2θ as follows: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°. Its pattern is shown in FIG. 1, which is the crystal form VI of the compound.
(2) The off-white solid powder prepared in step (2) above was identified by TA Q200 differential scanning calorimetry (DSC) analysis: the scanning speed was 10°C/min, and an endothermic peak at 227.0°C (with a melting point onset peak at 218.5°C) was included, with an error margin of ±3°C. Its pattern is shown in FIG. 2.
(3) The off-white solid powder prepared in step (2) above was identified by TA Q500 thermogravimetric analysis (TGA): the scanning speed was 10°C/min, and its weight loss of 0.21% between 30°C and 150°C was shown. Its pattern is shown in FIG. 3.

### Embodiment 2

### 1. Preparation of Crystal Form VI by Evaporative Crystallization in Dichloromethane/Acetone System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 30.0 g of the compound raw material was added to dichloromethane (150 mL) and stirred at room temperature for dissolution. Then, 4/5 of the dichloromethane solution was concentrated under reduced pressure. Acetone (300 mL) was slowly added dropwise at a controlled temperature of 40°C to induce crystallization. Then, 1/2 of the solution was continued to be concentrated under reduced pressure. The mixture was then cooled to 20°C, stirred for 1 h, subjected to suction filtration, and dried to obtain 27.9 g of an off-white solid powder with a yield of 93.0%. XRPD measurement confirmed it as a crystal form VI.

### 2. Identification of Crystal Form VI

(1) The off-white solid powder prepared in step (2) above was identified by Bruker D8 Advance X-ray powder diffraction (XRPD) analysis: Cu-Kα radiation was used, and it had characteristic peaks represented by the angle 2θ as follows: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°. Its pattern was substantially consistent with that shown in FIG. 1.
(2) The off-white solid powder prepared in step (2) above was identified by TA Q200 differential scanning calorimetry (DSC) analysis: the scanning speed was 10°C/min, and an endothermic peak at 227.0°C (with a melting point onset peak at 218.5°C) was included, with an error margin of ±3°C. Its pattern was substantially consistent with that shown in FIG. 2.
(3) The off-white solid powder prepared in step (2) above was identified by TA Q500 thermogravimetric analysis (TGA): the scanning speed was 10°C/min, and its weight loss of 0.21% between 30°C and 150°C was shown. Its pattern was substantially consistent with that shown in FIG. 3.

### Embodiment 3

### 1. Preparation of Crystal Form VI by Volatilization Crystallization in Acetone/Isopropyl Acetate System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 100 mg of the compound raw material was added to acetone (5 mL) and isopropyl acetate (1.5 mL), and heated to 50°C for complete dissolution to obtain a clear solution. The mixture was then left open to slowly volatilize the solvent, resulting in the precipitation of a solid. The solid was dried to obtain 83 mg of an off-white solid powder with a yield of 83%. XRPD measurement confirmed it as a crystal form VI.

### 2. Identification of Crystal Form VI

(1) The off-white solid powder prepared in step (2) above was identified by Bruker D8 Advance X-ray powder diffraction (XRPD) analysis: Cu-Kα radiation was used, and it had characteristic peaks represented by the angle 2θ as follows: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°. Its pattern was substantially consistent with that shown in FIG. 1.
(2) The off-white solid powder prepared in step (2) above was identified by TA Q200 differential scanning calorimetry (DSC) analysis: the scanning speed was 10°C/min, and an endothermic peak at 227.0°C (with a melting point onset peak at 218.5°C) was included, with an error margin of ±3°C. Its pattern was substantially consistent with that shown in FIG. 2.
(3) The off-white solid powder prepared in step (2) above was identified by TA Q500 thermogravimetric analysis (TGA): the scanning speed was 10°C/min, and its weight loss of 0.21% between 30°C and 150°C was shown. Its pattern was substantially consistent with that shown in FIG. 3.

### Embodiment 4

### 1. Preparation of Crystal Form VI by Slurry Conversion in Acetone System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 2.0 g of the compound raw material was added to acetone (30 mL) and stirred as a suspension at 50°C for 12 h. The mixture was then slowly cooled to 0°C, stirred for 5 h, subjected to suction filtration, and dried to obtain 1.88 g of an off-white solid powder with a yield of 94.0%. XRPD measurement confirmed it as a crystal form VI.

### 2. Identification of Crystal Form VI

(1) The off-white solid powder prepared in step (2) above was identified by Bruker D8 Advance X-ray powder diffraction (XRPD) analysis: Cu-Kα radiation was used, and it had characteristic peaks represented by the angle 2θ as follows: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°. Its pattern was substantially consistent with that shown in FIG. 1.
(2) The off-white solid powder prepared in step (2) above was identified by TA Q200 differential scanning calorimetry (DSC) analysis: the scanning speed was 10°C/min, and an endothermic peak at 227.0°C (with a melting point onset peak at 218.5°C) was included, with an error margin of ±3°C. Its pattern was substantially consistent with that shown in FIG. 2.
(3) The off-white solid powder prepared in step (2) above was identified by TA Q500 thermogravimetric analysis (TGA): the scanning speed was 10°C/min, and its weight loss of 0.21% between 30°C and 150°C was shown. Its pattern was substantially consistent with that shown in FIG. 3.

### Embodiment 5

### 1. Preparation of Crystal Form VI by Slurry Conversion in Acetonitrile System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 1.0 g of the compound raw material was added to acetonitrile (10 mL) and stirred as a suspension at 25°C for 24 h. The mixture was subjected to suction filtration and dried to obtain 0.89 g of an off-white solid powder with a yield of 89.0%. XRPD measurement confirmed it as a crystal form VI.

### 2. Identification of Crystal Form VI

(1) The off-white solid powder prepared in step (2) above was identified by Bruker D8 Advance X-ray powder diffraction (XRPD) analysis: Cu-Kα radiation was used, and it had characteristic peaks represented by the angle 2θ as follows: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°. Its pattern was substantially consistent with that shown in FIG. 1.
(2) The off-white solid powder prepared in step (2) above was identified by TA Q200 differential scanning calorimetry (DSC) analysis: the scanning speed was 10°C/min, and an endothermic peak at 227.0°C (with a melting point onset peak at 218.5°C) was included, with an error margin of ±3°C. Its pattern was substantially consistent with that shown in FIG. 2.
(3) The off-white solid powder prepared in step (2) above was identified by TA Q500 thermogravimetric analysis (TGA): the scanning speed was 10°C/min, and its weight loss of 0.21% between 30°C and 150°C was shown. Its pattern was substantially consistent with that shown in FIG. 3.

### Comparative Example 1

### 1. Preparation of Compound

Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained.

### 2. Identification of Compound

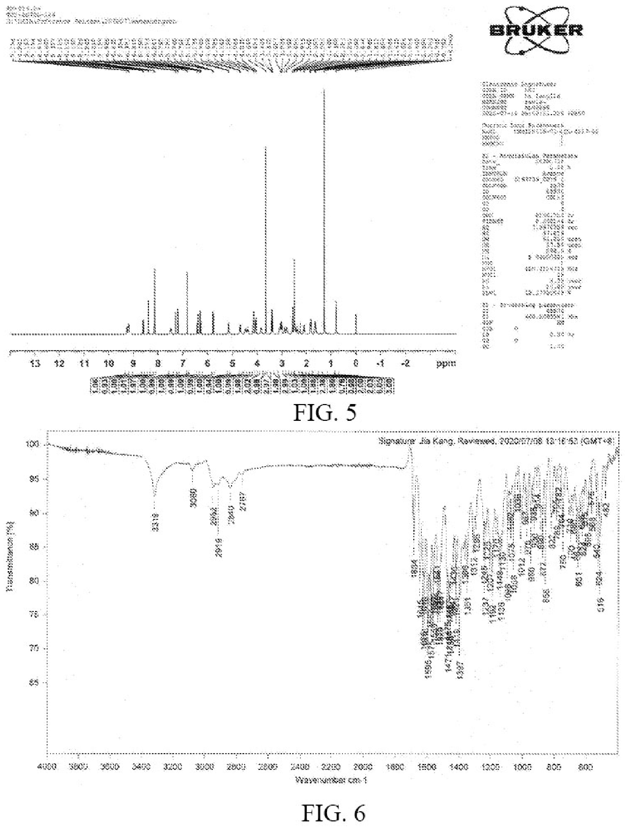

An appropriate quantity of the compound solid from step 1 was taken and tested for XRPD. Its pattern is shown in FIG. 4, which is amorphous. The proton nuclear magnetic resonance spectrum and infrared spectrum of the compound from step 1 were determined. The results showed that both the proton nuclear magnetic resonance spectrum and the infrared spectrum were consistent with the structure of the target compound. The proton nuclear magnetic resonance spectrum is shown in FIG. 5, and the specific data of the proton nuclear magnetic resonance spectrum are shown in Table 2 below. The infrared spectrum is shown in FIG. 6, and the specific data of the infrared spectrum are shown in Table 3 below.

**Table 2**

| **¹H chemical shift (ppm, TMS)** | **Number of protons** | **Multiplicity (*J* in Hz)*** | **Assignment** |
|---|---|---|---|
| 9.25, 9.20** | 1 | s, s | 6 |
| 9.18, 9.17** | 1 | s, s | 20 |
| 8.61 | 1 | d*, J =* 5.2 | 36 |
| 8.38 | 1 | s | 25 |
| 8.17-8.13 | 2 | m | 35 |
| | | s | 29 |
| 7.51-7.45 | 1 | m | 2 |
| 7.20 | 1 | d, *J* = 8.4 | 3 |
| 6.83 | 1 | s | 51 |
| 6.44-6.39 | 1 | m | 24a |
| 6.32-6.25 | 1 | dd, *J =* 10.0, 10.4 | 22 |
| 5.79-5.76 | 1 | dd, *J =* 10.0, 1.2 | 24b |
| 5.15 | 1 | *d, J =* 12.8 | 41 |
| 4.69 | 1 | *d, J =* 12.0 | 40a |
| 4.52-4.46 | 1 | m | 43b |
| 4.39 | 1 | t, *J =* 11.2 | 40b |
| 4.14 | 2 | t, *J* = 5.6 | 44 |
| 4.07-4.04 | 2 | dd, *J =* 10.8, 3.2 | 16a, 18a |
| 3.84 | 1 | *d, J =* 11.2 | 43b |
| 3.65 | 3 | s | 33 |
| 3.40 | 2 | t, *J* = 11.6 | 16b, 18b |
| 3.10-2.99 | 3 | m | 8, 9a, 11a |
| 2.92-2.87 | 1 | m | 12a |
| 2.82 | 1 | m | 12b |
| 2.56 | 2 | d, *J* = 5.2 | 52 |
| 2.50-2.44 | 2 | s | 54 |
| | 1 | m | 14 |
| 2.41-2.36 | 1 | m | 11b |
| 2.09 | 1 | t, *J* = 10.0 | 9b |
| 1.84-1.81 | 2 | m | 15a, 19a |
| 1.68-1.57 | 2 | m | 15b, 19b |
| 1.26 | 6 | s | 55, 56 |
| 0.79 | 3 | d, *J* = 5.2 | 13 |

| | | | |
|---|---|---|---|
| Note: m is a multiplet; s is a singlet; d is a doublet; t is a triplet; and dd is a doublet of doublets. | | | |

**Table 3**

| **Absorption peak (cm⁻¹)** | **Absorption intensity** | **Vibration type** | **Assignment** |
|---|---|---|---|
| 3319 | w | O-H and N-H stretching vibrations | Alcoholic hydroxyl O-H and amino N-H |
| 3080 | w | C-H stretching vibration | Aromatic ring C-H |
| 2952-2840 | w | C-H stretching vibration | Alkyl C-H |
| 1684, 1645, 1625-1616 | s | C=O stretching vibration | Amide C=O |
| 1595, 1582, 1533-1447 | s | C=C and C=N stretching vibrations | Aromatic ring C=C and heteroaromatic ring C=N |
| 1397, 1351, 1296 | s | C-H bending vibration | Alkyl C-H and aromatic amine C-N |
| | | C-N stretching vibration | |
| 1237 | m | C-N stretching vibration | Amide C-N |
| 1192, 1138 | s | C-C and C-O stretching vibrations | Alkyl C-C and aliphatic ether C-O |
| 1058, 1012 | s | C-N and C-O stretching vibrations | Amino C-N and alcoholic hydroxyl C-O |
| 858, 822 | s | C-H bending vibration | Aromatic ring C-H |

| | | | |
|---|---|---|---|
| Note: m represents medium, s represents strong, and w represents weak. | | | |

### Comparative Example 2

### 1. Preparation of Crystal Form V by Crystallization in Trifluoroethanol/Water System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 1.0 g of the compound raw material was added to trifluoroethanol (3 mL) and stirred at 50°C for complete dissolution to obtain a clear solution. Pure water (8 mL) was then slowly added dropwise to induce crystallization. Stirring was continued at 50°C for 18 h, followed by suction filtration. After air-drying at room temperature for 4 h, 0.95 g of an off-white solid powder was obtained with a yield of 95.0%. XRPD measurement confirmed it as a crystal form V.

### 2. Identification of Crystal Form V

The off-white solid powder prepared in step (2) above was identified by Bruker D8 Advance X-ray powder diffraction (XRPD) analysis: Cu-Kα radiation was used, and it had characteristic peaks represented by the angle 2θ as follows: 4.1±0.2°, 9.2±0.2°, 11.7±0.2°, 14.8±0.2°, 15.4±0.2°, 18.2±0.2°, 19.9±0.2°, 20.5±0.2°, 21.5±0.2°, 22.2±0.2°, 25.0±0.2°, and 27.7±0.2°. Its pattern is shown in FIG. 7, which is the crystal form V.

### Comparative Example 3

### 1. Preparation of Crystal Form XIII by Slurry Conversion in Dimethyl Sulfoxide System

(1) Referring to the preparation method disclosed in Patent Publication No. CN111741959A, the compound, (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide, was obtained (the solid form is not limited);
(2) 1.5 g of the compound raw material was added to dimethyl sulfoxide (18 mL) and stirred as a suspension at 25°C for 24 h. The mixture was subjected to suction filtration and dried to obtain 1.34 g of an off-white solid powder with a yield of 89.3%. XRPD measurement confirmed it as a crystal form XIII.

### 2. Identification of Crystal Form XIII

(1) The off-white solid powder prepared in step (2) above was identified by Bruker D8 Advance X-ray powder diffraction (XRPD) analysis: Cu-Kα radiation was used, and it had characteristic peaks represented by the angle 2θ as follows: 11.5±0.2°, 14.5±0.2°, 14.8±0.2°, 15.1±0.2°, 16.9±0.2°, 17.6±0.2°, 18.8±0.2°, 19.3±0.2°, 20.0±0.2°, 20.8±0.2°, 21.5±0.2°, and 24.3±0.2°. Its pattern is shown in FIG. 8, which is the crystal form XIII.
(2) The off-white solid powder prepared in step (2) above was identified by TA Q200 differential scanning calorimetry (DSC) analysis: the scanning speed was 10°C/min, and two endothermic peaks at 129.0°C and 261.5°C were included, with an error margin of ±3°C. Its pattern is shown in FIG. 9.
(3) The off-white solid powder prepared in step (2) above was identified by TA Q500 thermogravimetric analysis (TGA): the scanning speed was 10°C/min, and its weight loss of about 14.71% between 30°C and 170°C was shown. Its pattern is shown in FIG. 10.

### Test Example 1 Dynamic Vapor Sorption Experiment

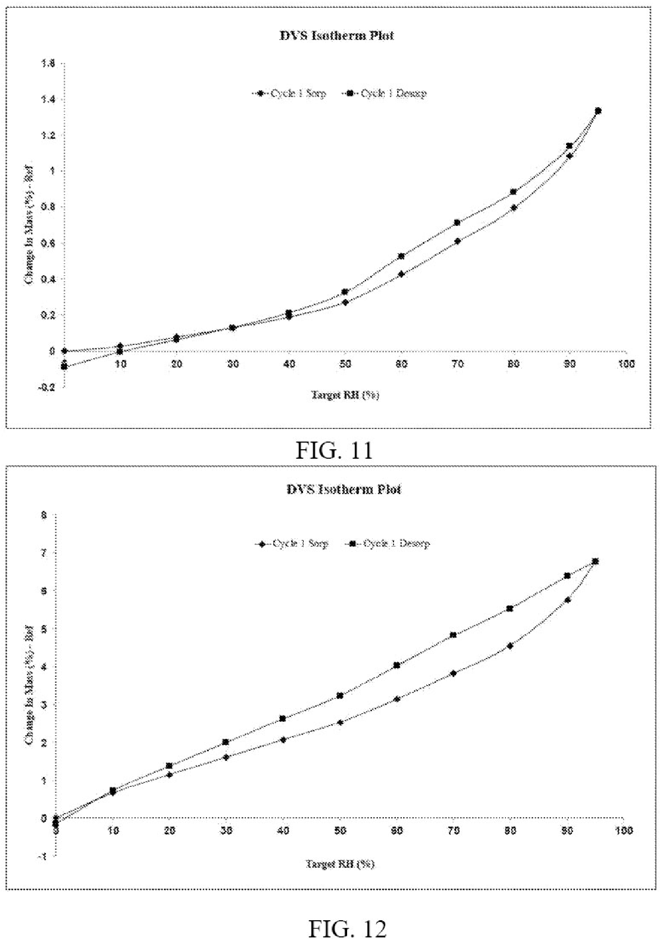

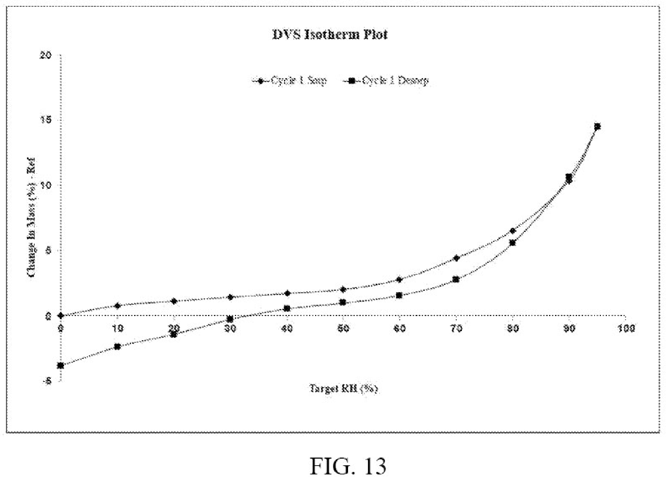

An appropriate quantity of the crystal form VI prepared in Example 1, the amorphous form prepared in Comparative Example 1, and the crystal form XIII prepared in Comparative Example 3 were respectively taken and subjected to dynamic vapor sorption (DVS) testing. Table 4 shows the DVS experimental results for the crystal form VI, the amorphous form, and the crystal form XIII. As can be seen from Table 4: the crystal form VI exhibited a weight gain of about 0.79% at 80% RH and about 1.34% at 95% RH (see FIG. 11, with an error margin of ±0.1% in the mass change), showing that it is slightly hygroscopic. In contrast, the amorphous form exhibited a weight gain of about 4.55% at 80% RH and about 6.76% at 95% RH (see FIG. 12), and the crystal form XIII exhibited a weight gain of about 6.53% at 80% RH and about 14.45% at 95% RH (see FIG. 13), showing that both are hygroscopic. This indicates that the crystal form VI has lower hygroscopicity, which is beneficial for the long-term storage of the drug.

**Table 4**

| **Crystal form** | **Adsorption weight gain (80% RH)** | **Adsorption weight gain (95% RH)** | **Hygroscopicity** |
|---|---|---|---|
| Crystal form VI | 0.79% | 1.34% | Slightly hygroscopic |
| Amorphous form | 4.55% | 6.76% | Hygroscopic |
| Crystal form XIII | 6.53% | 14.45% | Hygroscopic |

Regarding the description of hygroscopicity characteristics and the definition of hygroscopic weight gain (Guiding Principles for Drug Hygroscopicity Testing, Appendix to the Chinese Pharmacopoeia 2010 Edition; experimental conditions: 25±0.2°C, 80% relative humidity):
Deliquescent: absorbs sufficient moisture to form a liquid;
Highly hygroscopic: hygroscopic weight gain is not less than 15%;
Hygroscopic: hygroscopic weight gain is less than 15% but not less than 2%;
Slightly hygroscopic: hygroscopic weight gain is less than 2% but not less than 0.2%; and
Non-hygroscopic or almost non-hygroscopic: hygroscopic weight gain is less than 0.2%.

### Test Example 2 Stability Test in Pure Water and Buffer Solution

The crystal form VI, the amorphous form, the crystal form V, and the crystal form XIII of the compound were formed into suspensions in pure water and buffer solutions of different pH values, respectively. The suspensions were stirred at 25°C for 24 h, and filtered to obtain the solids. Samples were taken for XRPD measurement. The specific experimental results are shown in Table 5 below. Table 5 shows the stability experiments of each crystal form in pure water and buffer solutions. As can be seen from Table 5, the crystal form of the crystal form VI remained unchanged after suspension in pure water and buffer solutions at 25°C for 24 h. In contrast, the amorphous form, the crystal form V, and the crystal form XIII transformed into other crystal forms after suspension in pure water and buffer solutions at 25°C for 24 h. This indicates that the crystal form VI has better stability.

**Table 5**

| **Starting crystal form** | **Solvent** | **Temperature** | **Suspension result** |
|---|---|---|---|
| Crystal form VI | Pure water | 25°C | Crystal form VI |
| | pH 4.5 buffer solution | 25°C | Crystal form VI |
| | pH 6.8 buffer solution | 25°C | Crystal form VI |
| Amorphous form | Pure water | 25°C | Transform into other crystal forms |
| | pH 4.5 buffer solution | 25°C | Transform into other crystal forms |
| | pH 6.8 buffer solution | 25°C | Transform into other crystal forms |
| Crystal form V | Pure water | 25°C | Transform into |
| | | | other crystal forms |
| | pH 4.5 buffer solution | 25°C | Transform into other crystal forms |
| | pH 6.8 buffer solution | 25°C | Transform into other crystal forms |
| Crystal form XIII | Pure water | 25°C | Transform into other crystal forms |
| | pH 4.5 buffer solution | 25°C | Transform into other crystal forms |
| | pH 6.8 buffer solution | 25°C | Transform into other crystal forms |

### Test Example 3 Influencing Factor Stability Test

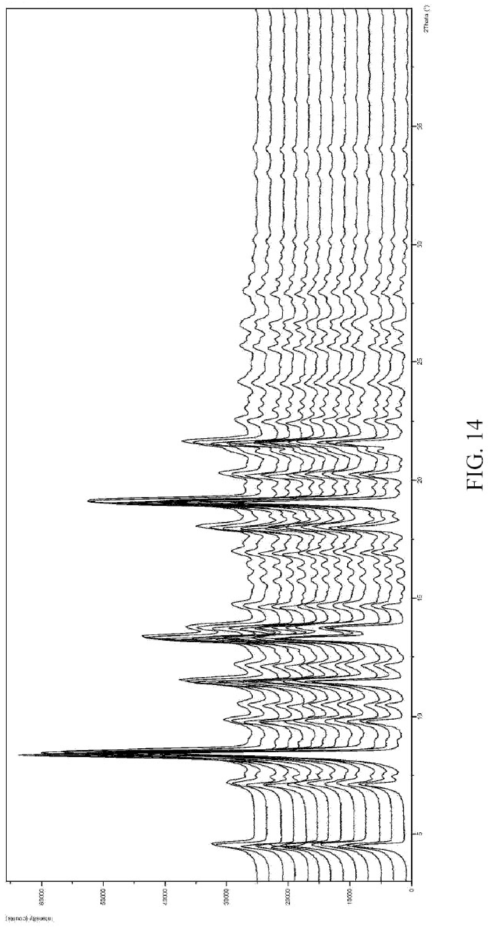

According to the guiding principles for stability tests of drug preparations, influencing factor experiments were performed on the crystal form VI, the amorphous form, and the crystal form XIII of the compound, including a high-temperature test, a high-humidity test, and a highlight exposure test, to investigate the factors affecting the stability of the above crystal forms. The crystal form results under different conditions were identified by XRPD analysis, and the purity results under different conditions were identified by HPLC analysis. The results for the crystal form VI are shown in FIG. 14 (in the figure, from low to high intensity, the patterns correspond to day 0, high temperature day 5, high temperature day 10, high temperature day 15, high temperature day 30, high humidity day 5, high humidity day 10, high humidity day 15, high humidity day 30, light exposure day 5, light exposure day 10, light exposure day 15, and light exposure day 30, respectively).

### (1) High-humidity test

Appropriate quantities of samples of the crystal form VI, the amorphous form, and the crystal form XIII were taken and spread flat in weighing bottles, and placed in a constant temperature and humidity chamber at 25°C and RH 92.5±5%. The above samples were then taken on days 5, 10, 15, and 30, respectively. Their crystal forms were tested by adopting X-ray powder diffraction (XRPD), and the sample purities were tested by adopting HPLC. Table 6 shows the high-humidity test results of the crystal form VI, the amorphous form, and the crystal form XIII. As can be seen from Table 6: after being placed under conditions of 25°C and 92.5±5% RH for 30 days, the crystal form of the crystal form VI remained unchanged, and no significant decrease in purity was observed. In contrast, after being placed under conditions of 25°C and 92.5±5% RH for 30 days, the crystal forms of the amorphous form and the crystal form XIII also did not transform, but the purities decreased to varying degrees. This indicates that the crystal form VI has better stability.

**Table 6**

| **Starting crystal form** | **Stability placement conditions** | **Placement time** | **Crystal form** | **Purity** |
|---|---|---|---|---|
| Crystal form VI | Temperature 25°C, relative humidity 92.5±5% | 5 days | Crystal form VI | 99.35% |
| | | 10 days | Crystal form VI | 99.35% |
| Purity 99.32% | | 15 days | Crystal form VI | 99.32% |
| | | 30 days | Crystal form VI | 99.31% |
| Amorphous form | Temperature 25°C, relative humidity 92.5±5% | 5 days | Amorphous form | 98.72% |
| | | 10 days | Amorphous form | 98.50% |
| Purity 98.80% | | 15 days | Amorphous form | 98.38% |
| | | 30 days | Amorphous form | 98.21% |
| Crystal form XIII | Temperature 25°C, relative humidity 92.5±5% | 5 days | Crystal form XIII | 98.92% |
| | | 10 days | Crystal form XIII | 98.86% |
| Purity 99.01% | | 15 days | Crystal form XIII | 98.81% |
| | | 30 days | Crystal form XIII | 98.77% |

### (2) High-temperature test

Appropriate quantities of samples of the crystal form VI, the amorphous form, and the crystal form XIII were taken and spread flat in weighing bottles, and placed in a drying oven at 60°C. The above samples were then taken on days 5, 10, 15, and 30, respectively. Their crystal forms were tested by adopting X-ray powder diffraction (XRPD), and the sample purities were tested by adopting HPLC. Table 7 shows the high-temperature test results of the crystal form VI, the amorphous form, and the crystal form XIII. As can be seen from Table 7: after being placed under conditions of 60°C for 30 days, the crystal form of the crystal form VI remained unchanged, and the purity decreased slightly. After being placed under conditions of 60°C for 30 days, the crystal form of the amorphous form also did not transform, but the purity decreased relatively significantly. In contrast, the crystal form XIII had already undergone crystal transformation after being placed under conditions of 60°C for 5 days, and the purity also decreased relatively significantly after being placed for 30 days. This indicates that the crystal form VI has better stability.

**Table 7**

| **Starting crystal form** | **Stability placement conditions** | **Placement time** | **Crystal form** | **Purity** |
|---|---|---|---|---|
| Crystal form VI | Temperature 60°C | 5 days | Crystal form VI | 99.31% |
| | | 10 days | Crystal form VI | 99.25% |
| Purity 99.32% | | 15 days | Crystal form VI | 98.96% |
| | | 30 days | Crystal form VI | 98.72% |
| Amorphous form | Temperature 60°C | 5 days | Amorphous form | 98.61% |
| | | 10 days | Amorphous form | 98.44% |
| Purity 98.80% | | 15 days | Amorphous form | 98.39% |
| | | 30 days | Amorphous form | 97.88% |
| Crystal form XIII | Temperature 60°C | 5 days | Transform into other crystal forms | 98.79% |
| | | 10 days | Transform into other crystal forms | 98.54% |
| Purity 99.01% | | 15 days | Transform into other crystal forms | 98.20% |
| | | 30 days | Transform into other crystal forms | 97.85% |

### (3) Light exposure test

Appropriate quantities of samples of the crystal form VI, the amorphous form, and the crystal form XIII were taken and spread flat in weighing bottles, and placed in a constant temperature and humidity chamber (25°C, RH 60%±5%) with visible light 4500 Lux±500 Lux and ultraviolet light 1.7 W*h/m². The above samples were then taken on days 5, 10, 15, and 30, respectively. Their crystal forms were tested by adopting X-ray powder diffraction (XRPD), and the sample purities were tested by adopting HPLC. Table 8 shows the light exposure test results of the crystal form VI, the amorphous form, and the crystal form XIII. As can be seen from Table 8: after being placed under conditions of visible light 4500 Lux±500 Lux and ultraviolet light 1.7 W*h/m² for 30 days, the crystal form of the crystal form VI remained unchanged, and the purity decreased slightly. After being placed under conditions of visible light 4500 Lux±500 Lux and ultraviolet light 1.7 W*h/m² for 30 days, the crystal forms of the amorphous form and the crystal form XIII also did not transform, but the purities decreased relatively significantly.

**Table 8**

| **Starting crystal form** | **Stability placement conditions** | **Placement time** | **Crystal form** | **Purity** |
|---|---|---|---|---|
| Crystal form VI Purity 99.32% | Visible light 4500 Lux ± 500 Lux, ultraviolet light 1.7 W*h/m² | 5 days | Crystal form VI | 99.17% |
| | | 10 days | Crystal form VI | 99.23% |
| | | 15 days | Crystal form VI | 98.71% |
| | | 30 days | Crystal form VI | 98.14% |
| Amorphous form Purity 98.80% | Visible light 4500 Lux ± 500 Lux, ultraviolet light 1.7 W*h/m² | 5 days | Amorphous form | 98.46% |
| | | 10 days | Amorphous form | 97.98% |
| | | 15 days | Amorphous form | 97.29% |
| | | 30 days | Amorphous form | 96.55% |
| Crystal form XIII Purity 99.01% | Visible light 4500 Lux ± 500 Lux, ultraviolet light 1.7 W*h/m² | 5 days | Crystal form XIII | 98.65% |
| | | 10 days | Crystal form XIII | 98.32% |
| | | 15 days | Crystal form XIII | 97.76% |
| | | 30 days | Crystal form XIII | 97.15% |

### Test Example 4 Accelerated Stability Test

Experimental process: the crystal form VI prepared in Example 1 was placed under accelerated stability conditions (40±2°C, RH=75±5%) for 1, 3, and 6 months, and then sampled for XRPD measurement. Table 9 shows the accelerated stability test results of the crystal form VI. As can be seen from Table 9: the crystal form VI remained unchanged after being placed under accelerated stability conditions for 6 months, exhibiting excellent physical and chemical stability.

**Table 9**

| **Placement conditions** | **40±2°C, 75±5 RH %** | | | |
|---|---|---|---|---|
| | **0 days** | **1 month** | **3 months** | **6 months** |
| Crystal form | Crystal form VI | Crystal form VI | Crystal form VI | Crystal form VI |
| Purity | 99.32% | 99.18% | 98.98% | 98.54% |

### Test Example 5 Long-Term Stability Test

Experimental process: the crystal form VI prepared in Example 1 was placed under long-term stability conditions (25±2°C, RH=60±5%) for 1, 6, 12, 18, and 24 months, and then sampled for XRPD measurement. Table 10 shows the long-term stability test results of the crystal form VI. As can be seen from Table 10 below: the crystal form VI remained unchanged after being placed under long-term stability conditions for 24 months, and no significant decrease in purity was observed, exhibiting excellent physical and chemical stability.

**Table 10**

| **Placement conditions** | **25±2°C, 60±5 RH %** | | | | | |
|---|---|---|---|---|---|---|
| | **0 days** | **1 month** | **6 months** | **12 months** | **18 months** | **24 months** |
| Crystal form | Crystal form VI | Crystal form VI | Crystal form VI | Crystal form VI | Crystal form VI | Crystal form VI |
| Purity | 99.32% | 99.31% | 99.35% | 99.28% | 99.31% | 99.30% |

### Test Example 6 Morphological Comparison Study of Crystal Form VI, Amorphous Form, and Crystal Form XIII

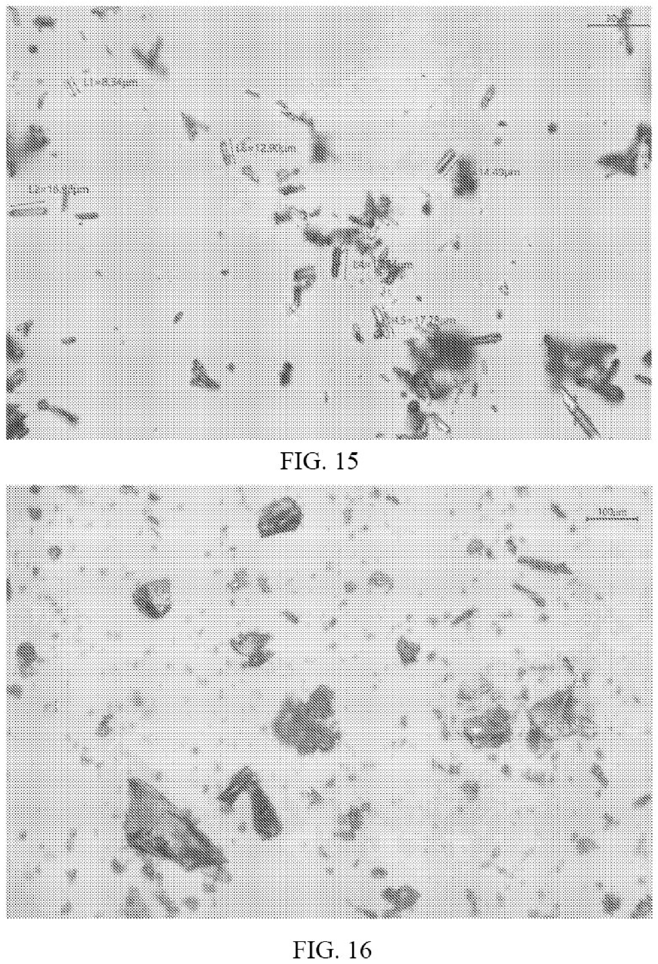

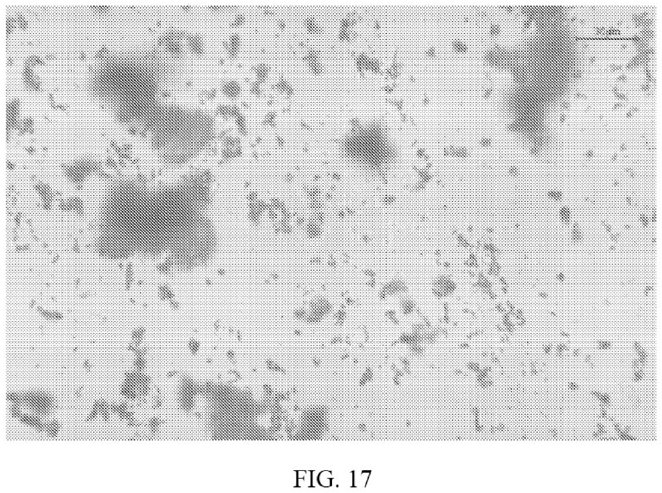

Experimental process: the crystal form VI prepared in Example 1, the amorphous form prepared in Comparative Example 1, and the crystal form XIII prepared in Comparative Example 3 were respectively photographed under a polarizing light microscope, as shown in FIGS. 15 to 17. As can be seen from FIGS. 15 to 17: from the photography results of the polarizing light microscope, the crystals of crystal form VI were long rod-shaped, with relatively good dispersibility and flowability. The amorphous solid was in an irregular glassy state, with significant agglomeration and caking. In contrast, the crystals of crystal form XIII were fine, also exhibiting agglomeration and caking, with relatively poor flowability.

### Test Example 7 Animal In Vivo PK Test of Crystal Form VI Tablets

Experimental process: with a basic formulation using the crystal form VI prepared in Example 1 as the bulk drug crystal form, microcrystalline cellulose as the filler, croscarmellose sodium as the disintegrant, magnesium stearate as the anti-adherent, and fumaric acid as the pH regulator, tablets were prepared for the animal *in vivo* PK test. Table 11 shows the animal *in vivo* PK experiment (beagle dogs, administration dosage of 10 mg/kg) of the crystal form VI preparation. As can be seen from Table 11: the tablet drug of crystal form VI showed good exposure in animals, meeting the exposure requirements.

**Table 11**

| | | | | | | |
|---|---|---|---|---|---|---|
| Oral in dogs (10 mg/kg) | | **Gender** | **Cₘₐₓ (ng•mL⁻¹)** | **Tₘₐₓ (h)** | **T_{1/2} (h)** | **AUC₀₋ₜ (ng•h•mL⁻¹)** |
| | PK (preparation) | Male (n=2) | 5310 | 1.50 | 2.52 | 22900 |
| | | Female (n=2) | 3535 | 1.0 | 2.95 | 21900 |

The above examples are preferred embodiments of the present invention, but the embodiments of the present invention are not limited by the above examples. Any other changes, modifications, substitutions, combinations, or simplifications made without departing from the spirit, essence and principles of the present invention shall be equivalent substitutions and are included within the scope of protection of the present invention.

## Claims

1. A crystal form VI of an (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide compound, wherein an X-ray powder diffraction pattern of the crystal form VI comprises diffraction peaks at the following 2θ angle values: 8.3±0.2°, 13.2±0.2° and 18.9±0.2°.

2. The crystal form VI according to claim 1, wherein the X-ray powder diffraction pattern of the crystal form VI comprises diffraction peaks at the following 2θ angle values: 8.3±0.2°, 13.2±0.2°, 13.6±0.2°, and 18.9±0.2°.

3. The crystal form VI according to claim 2, wherein the X-ray powder diffraction pattern of the crystal form VI comprises diffraction peaks at the following 2θ angle values: 8.3±0.2°, 11.3±0.2°, 13.2±0.2°, 13.6±0.2°, 18.9±0.2°, and 21.5±0.2°.

4. The crystal form VI according to claim 3, wherein the X-ray powder diffraction pattern of the crystal form VI comprises diffraction peaks at the following 2θ angle values: 4.3±0.2°, 8.3±0.2°, 11.3±0.2°, 13.2±0.2°, 13.6±0.2°, 17.8±0.2°, 18.9±0.2°, and 21.5±0.2°.

5. The crystal form VI according to claim 4, wherein the X-ray powder diffraction pattern of the crystal form VI comprises diffraction peaks at the following 2θ angle values: 4.3±0.2°, 6.9±0.2°, 8.3±0.2°, 9.7±0.2°, 11.3±0.2°, 13.2±0.2°, 13.6±0.2°, 14.5±0.2°, 17.8±0.2°, 18.9±0.2°, 20.1±0.2°, and 21.5±0.2°.

6. The crystal form VI according to claim 5, wherein the crystal form VI has an X-ray powder diffraction pattern substantially consistent with that shown in FIG. 1.

7. The crystal form VI according to claim 1, wherein the crystal form VI is an anhydrate.

8. The crystal form VI according to claim 1, wherein the crystal form VI has a purity of >85%; preferably, the crystal form VI has a purity of >95%; and preferably, the crystal form VI has a purity of >99%.

9. The crystal form VI according to any one of claims 1-8, wherein a melting point onset peak of the crystal form VI is at 218.5±3°C.

10. The crystal form VI according to claim 9, wherein the crystal form VI has a DSC pattern substantially consistent with that shown in FIG. 2.

11. A preparation method for the crystal form VI of the (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide compound according to any one of claims 1-10, comprising: adding the compound into a first solvent to dissolve the compound and form a clear solution, and then treating the clear solution using a crystallization process to obtain the crystal form VI, wherein the first solvent is selected from one or more of alcohol-based, carboxylic ester-based, nitrile-based, ketone-based, amide-based, sulfone-based, sulfoxide-based, carboxylic acid-based, nitro compound, pyridine-based, furan-based, alkylbenzene-based, and halogenated hydrocarbon-based polar solvents; or
adding the compound into a second solvent for slurrying, cooling, and separating a precipitated crystal form VI, wherein the second solvent is selected from one or more of alcohol-based, ether-based, alkane-based, carboxylic ester-based, nitrile-based, ketone-based, or alkylbenzene-based solvents.

12. The preparation method according to claim 11, wherein the crystallization process comprises performing crystallization by volatilization on the clear solution to volatilize the first solvent and obtain the crystal form VI.

13. The preparation method according to claim 12, wherein the first solvent is selected from ketone-based and/or carboxylic ester-based polar solvents; and
preferably, the first solvent is selected from a ketone-based polar solvent containing 3-6 carbon atoms and a carboxylic ester-based polar solvent containing 3-10 carbon atoms.

14. The preparation method according to claim 11, wherein the crystallization process comprises performing crystallization by evaporation on the clear solution to obtain the crystal form VI.

15. The preparation method according to claim 14, wherein the first solvent is selected from halogenated hydrocarbon-based and/or ketone-based polar solvents; and
preferably, the first solvent is selected from a halogenated hydrocarbon-based polar solvent containing 1-6 carbon atoms and a ketone-based polar solvent containing 3-6 carbon atoms.

16. The preparation method according to claim 11, wherein the crystallization process comprises adding a poor solvent to the clear solution to precipitate the crystal form VI.

17. The preparation method according to claim 16, wherein the first solvent is selected from one or more of amide-based, halogenated hydrocarbon-based, sulfoxide-based, and furan-based polar solvents; and the poor solvent is selected from one or more of an alcohol, a carboxylic ester, a nitrile, a ketone, an alkane, an ether, a nitro compound, and an alkylbenzene; and
preferably, the first solvent is selected from a halogenated hydrocarbon-based polar solvent, and the poor solvent is selected from an ether-based solvent.

18. The preparation method according to claim 11, wherein the second solvent is a ketone-based and/or nitrile-based solvent; and
preferably, the second solvent is a ketone-based solvent containing 3-6 carbon atoms or a nitrile-based solvent containing 2-6 carbon atoms.

19. The preparation method according to claim 11 or 18, wherein a volume-to-mass ratio of the second solvent to the compound is 1-30 mL/g.

20. A pharmaceutical composition, comprising a therapeutically effective amount of the crystal form VI of the compound according to any one of claims 1-10 and optionally a pharmaceutically acceptable carrier or adjuvant.

21. Use of the crystal form VI of the (S)-N-(5-((6-(2-(7,7-dimethyl-1-oxo-1,3,4,6,7,8-hexahydro-2H-cyclopenta[4,5]pyrrolo[1,2-a]pyrazin-2-yl)-3-(hydroxymethyl)pyridin-4-yl)-4-methyl-3-oxo-3,4-dihydropyrazin-2-yl)amino)-2-(2-methyl-4-(tetrahydro-2H-pyran-4-yl)piperazin-1-yl)phenyl)acrylamide compound according to any one of claims 1-10 or the pharmaceutical composition according to claim 17 in preparation of a medicament for preventing and/or treating a neoplastic disease or tumor, an autoimmune disease, and an inflammatory disorder.

22. The use according to claim 21, wherein the autoimmune disease and the inflammatory disorder are pemphigus vulgaris, rheumatoid arthritis, asthma, multiple sclerosis, systemic lupus erythematosus, or allergy.

23. The use according to claim 22, wherein the neoplastic disease or tumor is a B-cell malignancy or a solid tumor.

24. The use according to claim 23, wherein the neoplastic disease or tumor is chronic lymphocytic leukemia, small lymphocytic lymphoma, mantle cell lymphoma, diffuse large B-cell lymphoma, or multiple myeloma.
